# EUROPEAN PATENT APPLICATION

(11) **EP 2 327 405 A1**
(43) Date of publication of application: **01.06.2011**
(21) Application number: 10251945.1
(22) Date of filing: 17.11.2010
(51) Int. Cl.: A61K 31/353, A61P 9/06

(54) **Pharmaceutical composition comprising cinchonains 1a and 1b, process for preparing an epimeric mixture of cinchonains 1a and 1b, use and method for reverting/combating ventricular fibrillation**

(30) Priority: 17.11.2009 WO PCT/BR2009/000381
(71) Applicant: Laboratorio Catarinense S/A., 89204-001 Joinville, SC (BR)
(72) Inventor: Silva Filho, Ney Osvaldo, 89201-500, Joinville-SC (BR); Velasco, Irineu Tadeu, 05441-050 SP (BR); Scalabrini Neto, Augusto, 04024-020, Sao Paulo-SP (BR); Pianowski, Luiz Francisco, 12917-025, Bragança Paulista (BR)
(74) Representative: Senior, Janet

(57) **Abstract**

The present invention relates to the effects of certain cinchonains, especially the epimeric mixture of cinchonains Ia and Ib, on pathologies that involve alterations in the cardiovascular system, essentially in combating, preventing and reverting ventricular fibrillation in human beings and animals, especially for combating spontaneous or electric-stimulus-induced ventricular fibrillation, to revert spontaneous or electric-stimulus-induced ventricular fibrillations, for preventing ventricular fibrillation, for the treatment of ventricular fibrillation of any etiology or for the treatment of post-fibrillation to maintain normal cardiac rhythm.

The present invention further relates to methods for the synthesis of an epimeric mixture of the chemical substances cinchonain Ia and Ib.

The present invention also relates to the use of these chemical substances in reverting/combating ventricular fibrillation and/or in protecting against ventricular fibrillation. A pharmaceutical composition will be used for administering these chemical substances in human beings and animals.

## Description

### Field of the Invention

The present invention relates to the effects of the composition comprising cinchonains Ia and Ib on pathologies that involve alterations in the cardiovascular system, essentially in combating, preventing and reverting ventricular fibrillation in human beings and animals, especially for combating spontaneous or electric-stimulus-induced ventricular fibrillation, to revert spontaneous or electric-stimulus-induced ventricular fibrillations, for preventing ventricular fibrillation, for the treatment of ventricular fibrillation of any etiology or for the treatment of post-fibrillation to maintain normal cardiac rhythm.

The pharmaceutical composition of the invention can also be used in the veterinary field.

The present invention further relates to methods for the synthesis of an epimeric mixture of the chemical substances cinchonain Ia and Ib.

The present invention also relates to the use of these chemical substances in reverting/combating ventricular fibrillation and/or in protecting against ventricular fibrillation. A pharmaceutical composition will be used for administering these chemical substances in human beings and animals.

### Background of the Invention

It is known that ventricular fibrillation is the most important cause of sudden death, being present in more than 70% of the cases of cardiac arrest, especially in patients with coronary cardiopathy. The electrophysiological mechanism thereof involves great disorganization of the cardiac electric activity, with alterations in the conduction of the stimulus and in the ventricular repolarization which lead to the development of multiple reentrant circuits, which are called fibrillation "rotors". It often takes place as a final event of a cardiopathy.

The survival rate for this arrhythmia is still very small, less than 5%. Even subjects in whom a reversal to sinus rhythm is achieved, the rate of discharge from hospital is disappointing. Thus, there is a great interest in discovering and standardizing measures that increase the survival in this critical situation.

So far, the only treatment available for this arrhythmia is electric defibrillation. According to recommendations of the "American Heart Association", electric defibrillation should always be tried as a first treatment, and may later be associated to the use of pharmaceuticals. The great difficulty of this therapeutics lies in two factors: first, the defibrillation must be used only by trained people, even the semi-automatic models, and the cost thereof is very high; secondly, the time for defibrillation is critical, since with every minute the chance of an effective reversal is reduced by 10%. Thus, a simple measure, which can be used rapidly by people without specific training would be of great utility.

Another important factor is that, so far, there are no pharmaceuticals that could act in preventing this type of arrhythmia, so as to protect people who exhibit any risk factor for ventricular fibrillation.

Ventricular fibrillation is a pathology characterized by an uncoordinated and fatal series of very rapid and ineffective ventricular contractions, produced by multiple chaotic electric impulses.

From the electric point of view, ventricular fibrillation is similar to atrial fibrillation, presenting, however, a much more serious prognostic. In ventricular fibrillation, the ventricles quiver and do not contract in a coordinated manner. Since no blood is pumped from the heart, ventricular fibrillation represents a type of cardiac arrest and, if not treated immediately, is fatal.

The causes of ventricular fibrillation are the same as those of cardiac arrest. The most common one is the inadequate flow of blood to the myocardium due to coronary arterial disease or to myocardial infarction. Other causes include shock at very low levels of potassium in the blood (hypokalemia).

Ventricular fibrillation causes loss of conscience in a matter of seconds. If it is not treated, the subject generally exhibits convulsive crises and irreversible brain damage after approximately five minutes, since there is no supply of oxygen to the brain any longer. Then, death takes place.

### Brief description of the figures

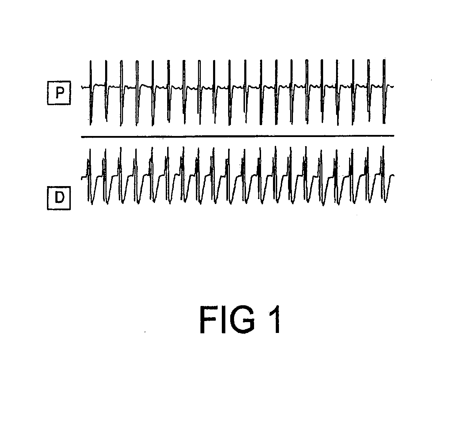

Figure 1 shows traces of P electrograms (register of the electrogram obtained from the proximal pair of electrodes) and D electrogams (register of the electrogram obtained from the distal pair of electrodes) of an insulated heart of rabbit.

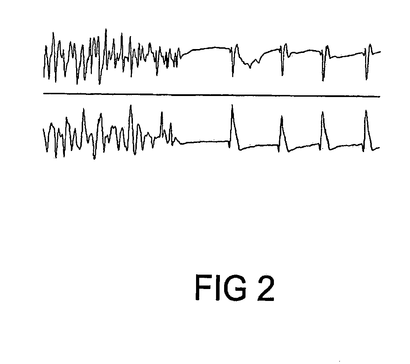

Figure 2 shows the reversal of ventricular fibrillation of the isolated heart of rabbit by endocardium stimulation with said epimeric mixture of cinchonains Ia and Ib.

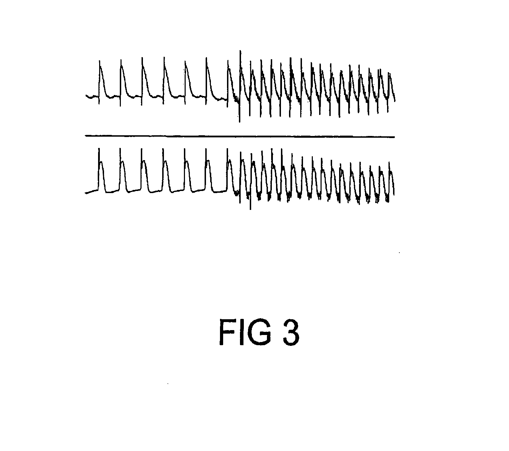

Figure 3 shows an attempt to reinduct ventricular fibrillation in the isolated heart of rabbit, in the presence of said epimeric mixture of cinchonains Ia and Ib. One cannot achieve new ventricular fibrillation with electric stimulus generated in the presence of said epimeric mixture of cinchonains I and Ib.

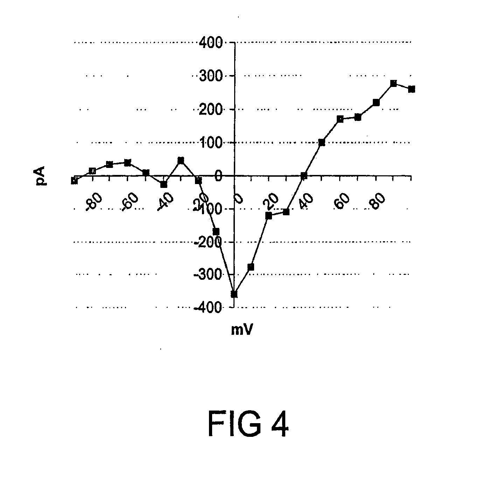

Figure 4 shows the results of the "patch-clamp" control experiment on isolated cell - isolated neuron). It shows that the sodium channels remain open.
Figure 5 shows the results of the "patch-clamp" experiments with said epimeric mixture of cinchonains I and Ib (200 µg/mL of the product or pharmaceutical composition of the present invention), time (T) = 0.
Figure 6 shows the results of the "patch-clamp" experiment with said epimeric mixture of cinchonains Ia and Ib (200 µg/mL of the product or pharmaceutical composition of the present invention), time (T) = 2 minutes.
Figure 7 shows the results of the "patch=clamp" experiment with said epimeric mixture of cinchonains Ia and Ib (200 µm/mL of the product or pharmaceutical composition of the present invention), time (T) = 5 minutes.

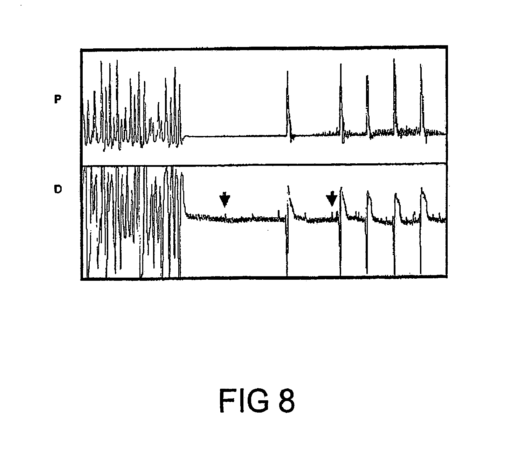

Figure 8 shows the reversal of sustained ventricular fibrillation in a typical experiment with said epimeric mixture of cinchonains Ia and Ib. There is reversal of ventricular fibrillation right after the addition of said epimeric mixture of cinchonains Ia and Ib to perfusate, with subsequent command of the supraventricular rhythm (probably sinus rhythm).

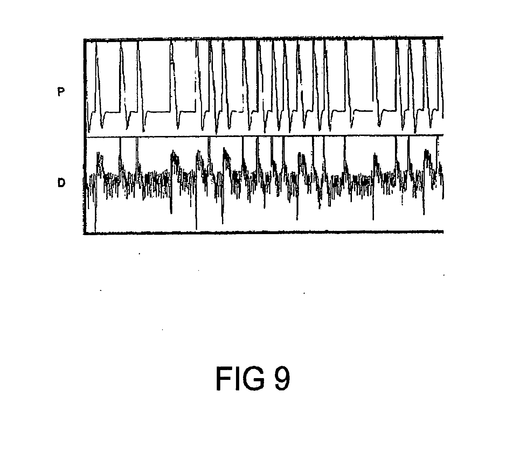

Figure 9 shows the attempt to reinduce ventricular fibrillation in a typical experiment with said epimeric mixture of cinchonains Ia and Ib. In spite of electric stimulation, no new ventricular fibrillation is developed.

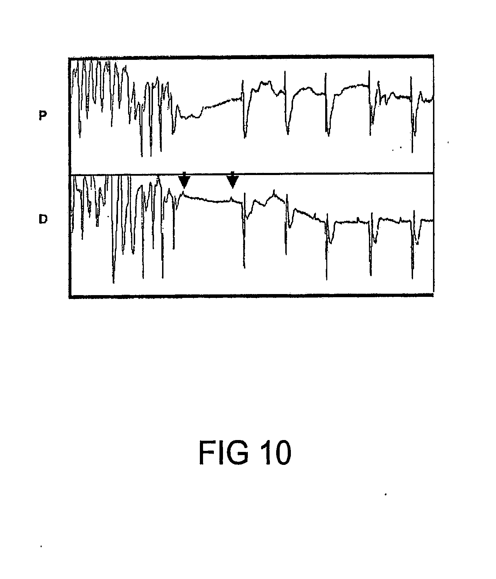

Figure 10 shows the reversal of sustained ventricular fibrillation in a typical experiment of said epimeric mixture of cinchonains Ia and Ib. There is reversal of ventricular fibrillation right after the addition of said epimeric mixture of cinchonains Ia and Ib to the perfusate, with subsequent command of supraventricular rhythm (probable sinus rhythm). The arrow marks the register of atrial electrogram.
Figure 11 shows the chemical reaction of the first synthesis process (Process I) of said epimeric mixture of cinchonains Ia and Ib.
Figure 12 shows the chemical reaction of the second synthesis process (Process II) of said epimeric mixture of cinchonains Ia and Ib.
Figures 13A to 13D show the combinations of radicals linked to said chemical substances.

### Summary of the Invention

The invention relates to pharmaceutical compositions comprising cinchonains Ia and Ib for reverting/combating and/or preventing ventricular fibrillation in human beings and animals, especially for combating spontaneous or electric-stimulus-induced ventricular fibrillation, to revert spontaneous or electric-stimulus-induced ventricular fibrillations, for preventing ventricular fibrillation, for the treatment of ventricular fibrillation of any etiology or for the treatment of post-fibrillation to maintain normal cardiac rhythm.

The pharmaceutical composition of the invention can also be used in the veterinary field.

The present invention further relates to methods for the synthesis of an epimeric mixture of the chemical substances Ia and Ib in reverting/combating and/or preventing ventricular fibrillation.

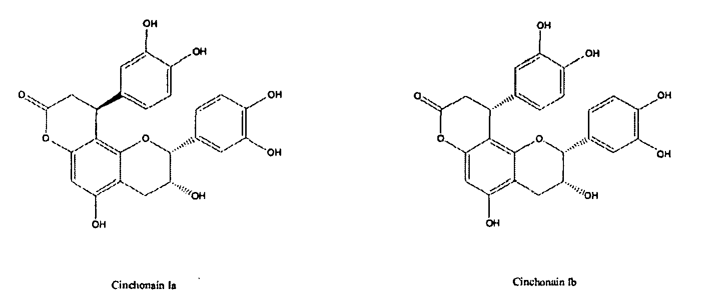

By "epimeric mixture of cinconains Ia and Ib" one should understand any one of the molecules obtained, either natural or synthetic, with any combination of radicals linked to these molecules, liable to produce equal or similar results in reverting/combating and/or preventing ventricular fibrillation.

In an embodiment, the invention relates to a method for reverting/combating and/or preventing ventricular fibrillation, using said substances, as well as to the use thereof in preparing compositions.

### Detailed Description of the Invention

The pharmaceutical composition of the invention, comprising cinchonain Ia and cinchonain Ib, in association with pharmaceutically acceptable carriers, is useful for combating/reverting ventricular fibrillation in human beings and animals, especially for combating spontaneous or electric-stimulus-induced ventricular fibrillation, to revert spontaneous or electric-stimulus-induced ventricular fibrillations, for preventing ventricular fibrillation, for the treatment of ventricular fibrillation of any etiology or for the treatment of post-fibrillation to maintain normal cardiac rhythm. Such substances are of fundamental importance when applied acutely, since ventricular fibrillation must be treated as an emergency and the only procedure available at present for reversal of ventricular fibrillation is electric shock onto the heart.

Even more important is the fact that, once the ventricular fibrillation has been reverted, recurrence thereof does not take place in the presence of said substances, a fact that does not occur with the procedure available at present of electric shock onto the heart.

The protection of people susceptible to ventricular fibrillation is of extreme importance, since it prolongs such people's life with quality and reduces the costs with interventions and hospitalization. The composition of the present invention exhibits efficacy in preventing ventricular fibrillation after chronic use of said chemical substances.

More particularly, one has found that cinchonains Ia and Ib have surprising effects on the reversal/combat and protection against ventricular fibrillation.

The compositions of the invention can be administered, but not limited to, for instance, oral administration in the form of tablets, coated tablets, hard or soft gelatin capsules, solutions, emulsions and suspensions; by rectal route in the form of suppositories, parenteral and intravenous administration, topical administration, in the transdermal, cutaneous form, among others.

Suitable carriers include, but are not limited to, lactose, starch or derivatives thereof, talc, stearic acid or its salts in the case of solid formulations for oral administration. On the other hand, the carriers suitable for soft gelatin capsules include plant oils, waxes, fats, semi-solid and liquid polyols. The solutions may be prepared comprising selected carriers such as water, polyols and carbohydrates. In the case of suppositories, the suitable carriers comprise natural or hardened oils, waxes, fats and polyols.

In addition to the carriers, the pharmaceutical compositions according to the present invention may contain preservatives, solubilizers, stabilizers, humectants, emulsifiers, sweeteners, colorants, flavoring agents, substances for adjusting tonicity, buffers, coating agents or antioxidants.

The compositions of the invention comprise from 5% to 95% by weight of the mixture/combination of cinchonains Ia and Ib. Preferably, the composition comprises from 25% to 95% by weight of the mixture/combination of cinchonain Ia and Ib.

The ratio of these components cinchonain Ia and Ib in an admixture of the invention may range for example from 1:10 to 10:1, and may be for example 1:1.

Although the invention is herein disclosed in more detail with respect to a mixture of chinchonains, the invention also relates to compositions comprising only one type of any chinchonains disclosed herein, as for example cinchonain 1 a or 1 b only or any mixture of chinchonains herein disclosed. The amounts when a single type of chinchonain is used should be those disclosed for the mixtures of cinchonains 1 a and 1 b

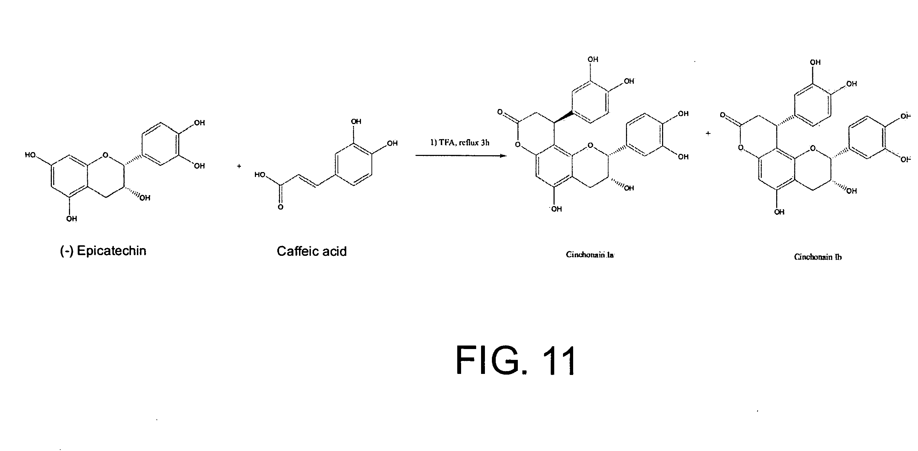

Thus, a compound for use in the present invention may comprise at least one of the cinchonains disclosed in Figures 11 to 13, and more especially the invention applies also to each of cinchonain 1 a and cinchonain 1 b, as well as to their admixture.

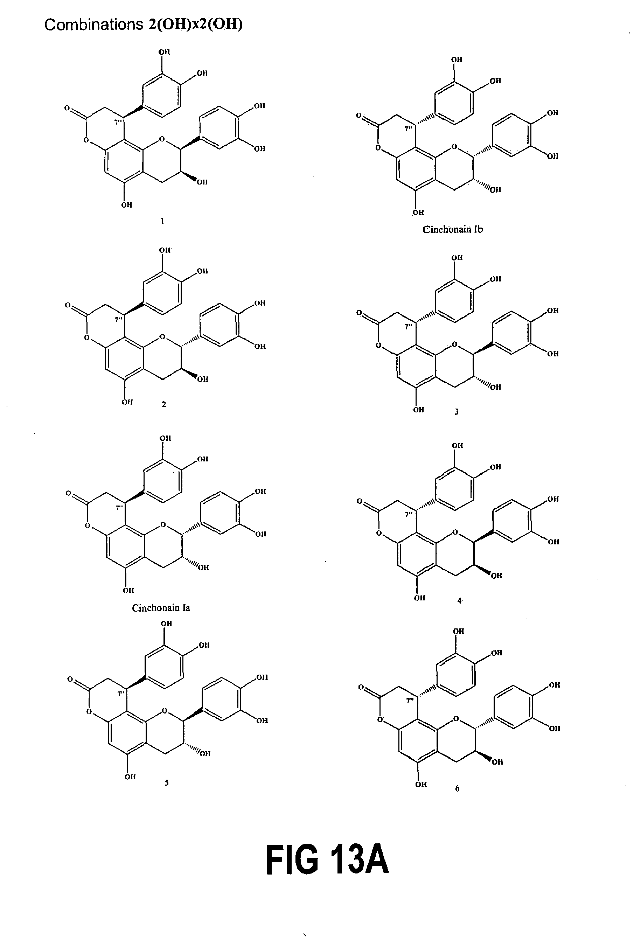

Figure 13 A shows a number of isomers of cinchonain 1 a and cinchonain 1 b, and the invention applies also to such isomers.

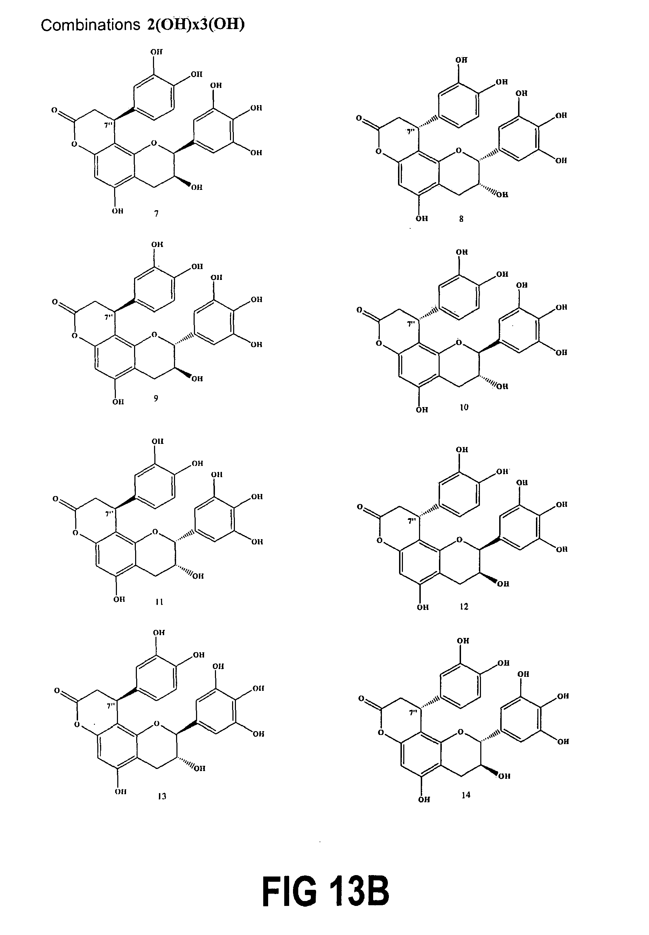

Cinchonains shown in Figures 13B to D having one or two additional hydroxy radicals should also be mentioned. The invention applies also to such hydroxy derivatives individually, and includes for example admixtures of cinchonain 1 a and cinchonain 1 b having the same additional hydroxy substitution, that is the same number of hydroxy radicals in the same positions.

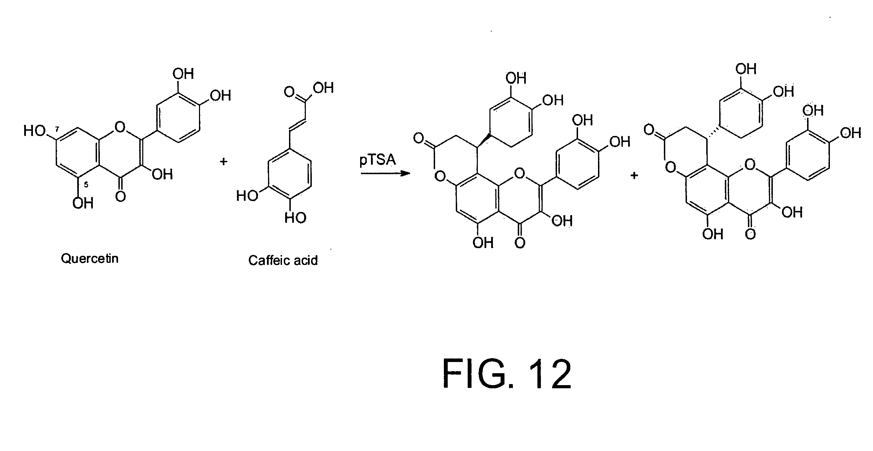

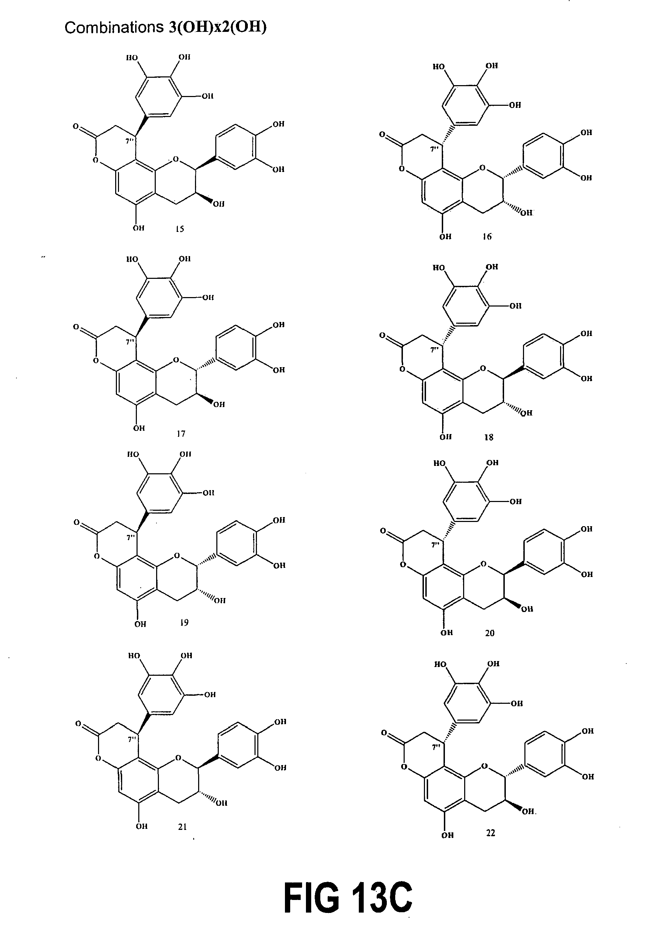

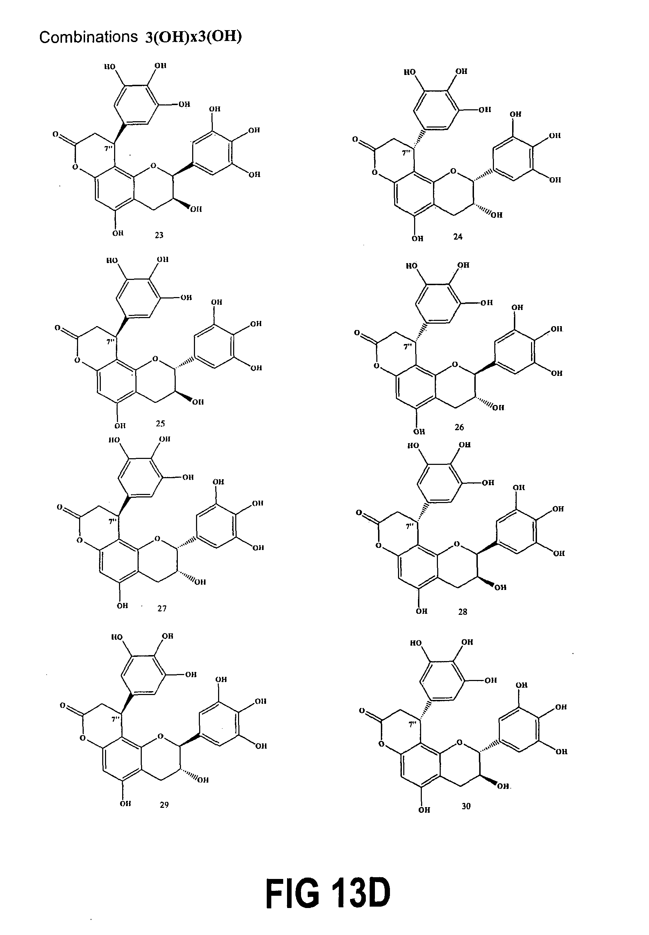

Thus, statements in the specification referring to a "mixture of cinchonain 1 a and cinchonain 1 b",and to "compositions according to the present invention", including statements referring to amounts, ratios and uses of the compounds, apply also to the individual compounds 1 a and 1 b, and to the individual end products shown in Figure 12 and to the individual compounds shown in Figs 13A, 13B, 13C and 13D, as well as, for example, to the Figure 12 (end product) admixture shown as well as admixtures of compounds of Fig 13. Thus the invention may use a single compound or a mixture.

The synthesis of an epimeric mixture of the chemical substances cinchonains Ia and Ib is obtained by chemical reaction of caffeic acid with epicatechin or quercitin, as shown below.

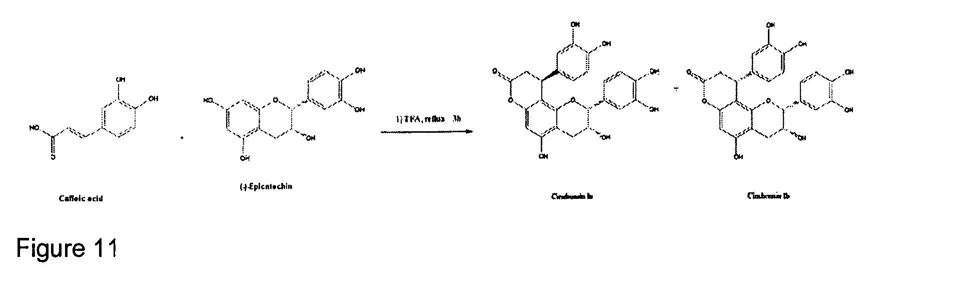

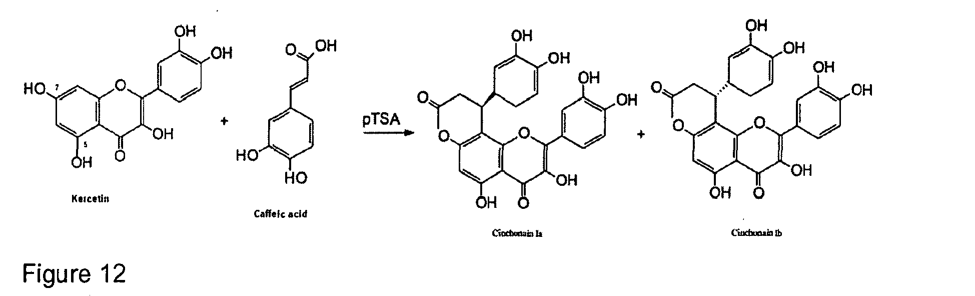

More particularly, the epimeric mixture of cinchonain Ia and Ib is obtained by the following processes:

### Process I:

The mixture of (-)epicatechin (1.0g - 3.44 mmol) and caffeic acid (910mg - 5.05 mmol) was dissolved in 30mL of trifluoroacetic acid (TFA) and kept under reflux for 3 hours. After cooling to room temperature, the reaction mixture was neutralized with 10% sodium bicarbonate, and extraction was made with ethyl acetate 3 x 50mL. Then, the reaction mixture was dried with anhydrous magnesium sulfate and the solvent was evaporated in Rotavapor. 873mg of sodium with brown color having a melting point of 240°C was obtained.

The mixture of cinchonains was then separated in preparatory HPLC (High Performance Liquid Chromatography) with yield higher than 70%.

### Process II:

In 50mL volumetric flask was added 1g of dehydrated quercetin, 0.5326g of caffeic acid, 0.56g of PTSA (p-toluenesulfonic acid) and 20mL of THF (tetrahydrofuran). The mixture was kept under stirring and heated up to 120°C for ± 3 hours. The reaction was accompanied by thin-layer chromatography (TLC) (80:20 ethyl acetate/hexane). The product was recrystallized in absolute ethanol. From the analysis of the nuclear magnetic resonance (NMR) ¹H NMR and ¹³C NMR, one concluded that the reaction between quercetin and caffeic acid led to the formation of the mixture of the isomers cinchonain Ia and Ib.

The mixture of cinchonains was then separated in preparatory HPLC with yield higher than 70%.

### Examples:

Studies and researches carried out now by the present inventors have demonstrated the efficacy of the composition comprising cinchonains Ia and Ib in combating/reverting and preventing ventricular fibrillation. In particular, said studies have demonstrated the efficacy thereof in reversal and protection against ventricular fibrillation related, particularly, to an epimeric mixture of cinchonains Ia and Ib, as shown by the data and tests described now in the present invention.

### Tests:

In order to prove the effect of said chemical substances on ventricular fibrillation in an isolated heart, the following study protocol has been carried out.

In order to carry out the experiments, twenty-seven male and female white New Zealand rabbits, weighing 2.5 - 3.5 kg, received 100 Ul/kg of intravenous heparin and were killed by cervical displacement.

The thorax was rapidly opened by middle-sized incision, and the sternum beaten back for complete exposure of the inside of the thorax. The pericardium was removed and the rising part of the aorta was isolated and repaired with a cotton thread 3-0. A metallic cannula was then inserted and fixed to the proximal portion of the aorta, retrograde perfusion of oxygenated Krebs-Henseleit solution was initiated and heated up to 37°C with constant flow of 13 mL/minute, kept by a continuous-infusion pump, Harvard Apparatus model RS232. The cannula tip was positioned quite close to the aortic valve to permit adequate coronary perfusion.

The heart was then removed and suspended through the cannula in a classic Langenhorff preparation. The Krebs Henseleit composition used was: 115 mM NaCl; 5.4 mM KCL; 1.25 mM CaCl₂; 25 mM NaHCO₃; 1.2 mM MgSO₄; 1.5 mM NaH₂PO₄ and 11 nM glucose. A drain was placed in the left ventricle (LV) to prevent accumulation of liquids.

Two pairs of electrodes (Monicron 2-O) were saturated on the LV: one at the tip, called proximal (P) and the other at the branching of front descending coronary artery branching with the first diagonal, called distal (D). The electrograms obtained from electrodes P and D were acquired through the ECG 100B acquisition system from Biopac System Inc. and registered continuously throughout the experiment through the software Acknowledge version 3.5.3, also from Biopac System Inc, with cut of frequency between 50 and 500 Hz. The cardiac frequency was determined by measuring the interval between two consecutive electrograms and transformed into beats per minute (bpm). The values considered were the average of 5 consecutive measurements.

For stabilization, the preparation was initially kept with open perfusion of Krebs solution, which perfused the coronaries and was discharged. After 20 minutes' stabilization, the system was closed and the perfusate was recirculated with 150 mL of solution.

A bipolar electrode was positioned and fixed at the endocardium surface of the right ventricle (RV) by incision in the pulmonary artery. A continuous electric stimulus was made through this electrode on the endocardium of the RV for 5 minutes, to trigger ventricular fibrillation. The stimulus was applied through the customized programmable stimulator. The electric stimulus had frequency of 50 Hz; duration of 2 ms; voltage of 10V and current of 10 mA.

After ventricular fibrillation kept for 20 minutes, a composition comprising cinchonains Ia and Ib was added to the persufate to test the anti-fibrillation effect and, in the cases where there was reversal, a new attempt was made to induce ventricular fibrillation after 5 minutes in order to determine the protective effect.

Said composition proved to be extremely effective in reverting sustained ventricular fibrillation induced in isolated heart of rabbit preparatory to classic Langdorff, with 100%-success. When compared with other classic pharmaceuticals, we can see that lidocain has a similar effect, whereas amiodarone and propranolol do not have any effect.

However, the re-induction of ventricular fibrillation in these same hearts (and, therefore, the action on the fibrillation threshold and, as a result, the prevention of ventricular fibrillation) was tested, we can see that lidocain does not have the same property. While said composition prevents ventricular fibrillation in all the hearts, lidocain does not prevent the re-induction, that is, it does not exhibit the same protective effect.

The mechanisms by which said chemical substances exert this effect are not completely understood yet, but it has been discovered that said composition administered acutely to the isolated heart of rabbit prolongs the time of conduction of the stimulus, apparently by blockage of the rapid sodium channel. This effect is very evident, but alone it does not justify integrally the anti-arrhythmia effect which is expected to be more dependent on the repolarization of the stimulus than on the conduction.

When one studies the repolarization, however, the results are surprising: said composition does not prolong the duration of the action potential (and, therefore, on the electrocardiogram (ECG), of the QTs interval), nor the time of recovery of the refractory period, that it, the time which the refractory period takes to recover after rapid stimulation.

When one measures the functional refractory period of the left ventricle (or relative refractory period, considering as the first stimulus conducted after extra-stimulus) and the effective refractory period of the left ventricle (or absolute refractory period, considering as the first beat blocked after extra-stimulus), one notes a clear and repetitive shortening of these measurements. Thus, said epimeric mixture cannot be classified as a proarrhythmia drug.

It is known that ventricular fibrillation depends on multiple reentrant circuits, or "rotors", to be sustained. The presence of reentrant circuits requires that the tissue onto which it is directed should have sufficient extension, and that the conduction of the stimulus and the ventricular repolarization should have critical velocity and duration. Especially the ventricular repolarization must compulsorily be shortened or asynchronous to maintain a compatible wave length.

Various drugs and substances alter the ventricular repolarization, leading to situation of lengthening of interval Q-T and, as a result, to malignant ventricular arrhythmias. Thus, the alterations of ventricular repolarization seem to perform a critical role in the development of these arrhythmias and possibly in the reversal thereof.

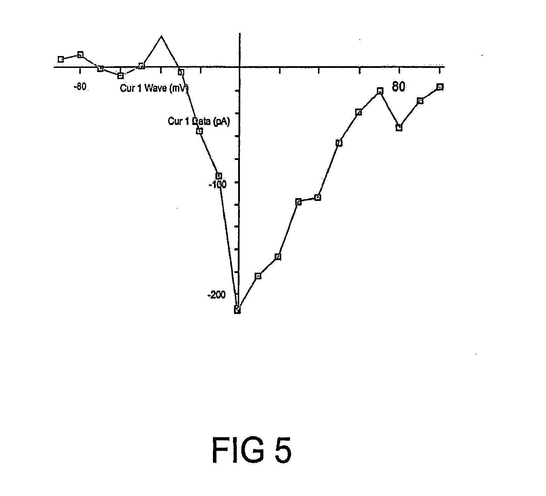

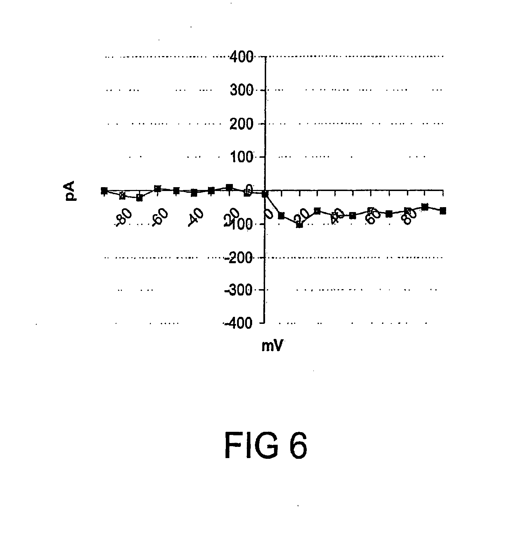

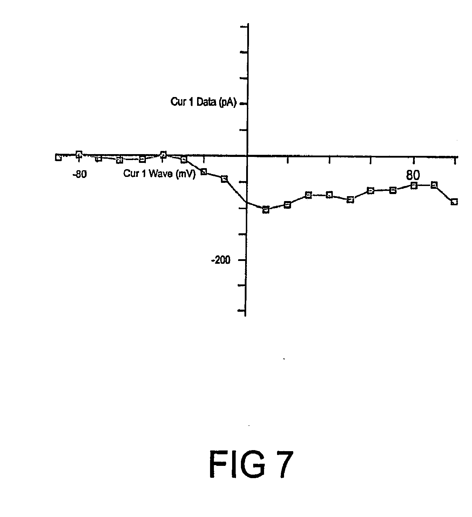

The results presented in figures 5, 6 and 7 show the inhibition of the sodium channel with the epimeric mixture of cinchonains Ia and Ib (9 µg/mL of the product or pharmaceutical composition of the present invention, times (T) 0, 2 and 5 minutes, keeping them closed).

The results described above evidence the fact that the composition now described in the present invention exhibits efficacy in reverting/combating and preventing ventricular fibrillation.

So far, there are no studies showing the effects of said chemical substances on the electrophysiological parameters of the heart. Thus, it is difficult to propose a mechanism for its anti-fibrillation effect. By similarity with other substances with anti-arrhythmic effect, one can speculate that the effect on the ventricular repolarization performs an important role in this action.

However, this is only an initial observation. Various other studies focusing on the effects of said chemical substances on the electrophysiological parameters of the heart and on the physiology of the membrane channel of the cardiomyocites should be carried out for better clarification of these mechanisms.

## Claims

1. A pharmaceutical composition, **characterized by** comprising cinchonain Ia and cinchonain Ib: 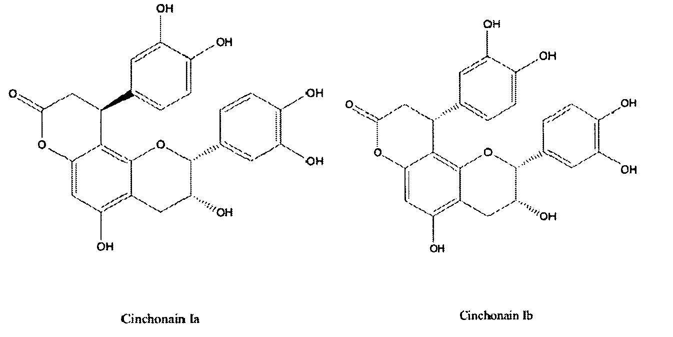 in association with pharmaceutically acceptable carriers, for combating spontaneous or electric-stimulus-induced ventricular fibrillation, to revert spontaneous or electric-stimulus-induced ventricular fibrillations, for preventing ventricular fibrillation, for the treatment of ventricular fibrillation of any etiology or for the treatment of post-fibrillation to maintain normal cardiac rhythm in human beings and animals.

2. A process for preparing an epimeric mixture of cinchonain Ia and Ib, **characterized by** comprising the chemical reaction of caffeic acid with epicatechin or quercitin.

3. A pharmaceutical composition, **characterized by** comprising a mixture obtained by the process as defined in claim 2, for combating spontaneous or electric-stimulus-induced ventricular fibrillation, to revert spontaneous or electric-stimulus-induced ventricular fibrillation, to prevent ventricular fibrillations, for the treatment of ventricular fibrillation of any etiology or for the treatment of post-fibrillation to maintain normal cardiac rhythm in human beings and animals.

4. The use of a composition as defined in claim 1, or of a mixture obtained by the process as defined in claim 2, **characterized by** being for the preparation of a composition for combating, reverting, treating and/or preventing ventricular fibrillation in human beings and animals.

5. The use according to claim 4, **characterized by** being for combating spontaneous or electric-stimulus-induced ventricular fibrillation.

6. The use according to claim 4, **characterized by** being for reversal of spontaneous or electric-stimulus-induced ventricular fibrillation.

7. The use according to claim 4, **characterized by** being for the treatment of ventricular fibrillation of any etiology.

8. The use of a composition as defined in claim 1, or of a mixture obtained by the process as defined in claim 2, **characterized by** being for the preparation of a composition for the treatment of post-fibrillation to maintain the normal cardiac rhythm in human beings and animals.

9. A method for reverting/combating ventricular fibrillation, **characterized in that** it comprises administering a composition as defined in claim 1, or of a mixture obtained by the process as defined in claim 2, to a human patient or animal in need of such treatment.

10. A pharmaceutical composition, **characterized by** comprising at least one of the cinchonains Ia or cinchonains Ib: 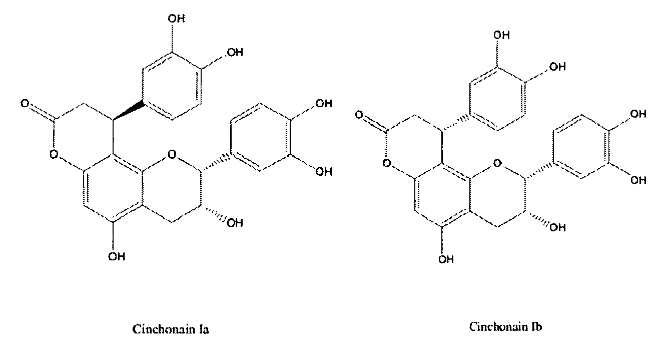 in association with pharmaceutically acceptable carriers, for combating spontaneous or electric-stimulus-induced ventricular fibrillation, to revert spontaneous or electric-stimulus-induced ventricular fibrillations, for preventing ventricular fibrillation, for the treatment of ventricular fibrillation of any etiology or for the treatment of post-fibrillation to maintain normal cardiac rhythm in human beings and animals.

11. A pharmaceutical composition for combating spontaneous or electrict-stimulus-induced ventricular fibrillation, to revert spontaneous or electric-stimulus-induced ventricular fibrillations, for preventing ventricular fibrillation, for the treatment of ventricular fibrillation of any etiology or for the treatment of post-fibrillation to maintain normal cardiac rhythm in human beings and animals; the composition containing at least one of the cinchonain Ia or cinchonain Ib as disclosed in the Figures 11,12 and/or 13.

12. Composition according to claims 10 or 11 wherein the composition comprises a mixture of cinchonains at a ratio ranging from 1:10 to 10:1.

13. Cinchonain 1 a or cinchonain 1 b 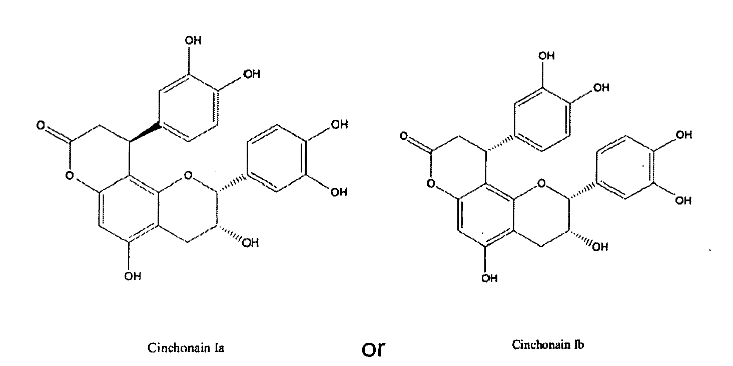 for use in combating, reversing, treating and/or preventing ventricular fibrillation, or for the treatment of post-fibrillation to maintain normal cardiac rhythm.

14. Any of the chinchonains disclosed in the Figures and drawings herein, for use in combating, reversing, treating and/or preventing ventricular fibrillation, or for the treatment of post-fibrillation to maintain normal cardiac rhythm.

15. A chinchonain as claimed in claim 14, of the formula 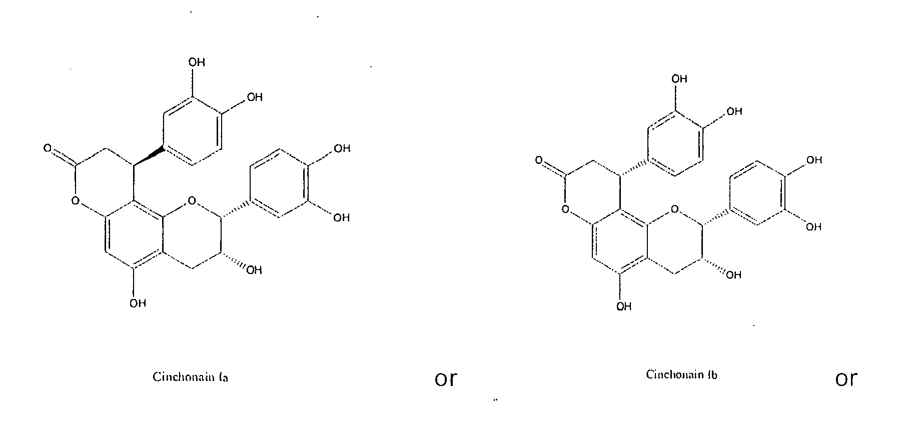 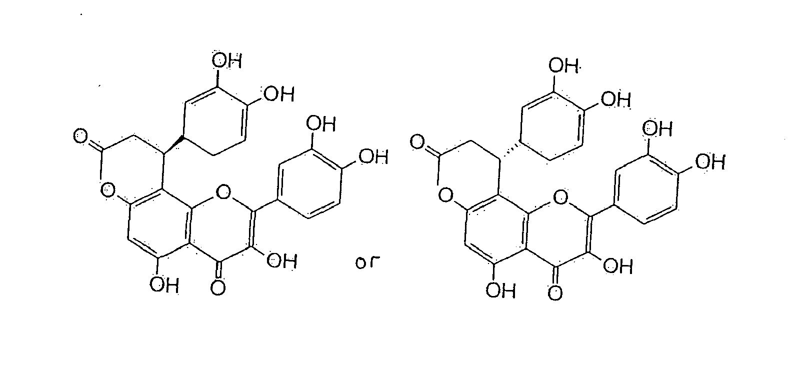 for use in combating, reversing, treating and/or preventing ventricular fibrillation, or for the treatment of post-fibrillation to maintain normal cardiac rhythm.
